# EUROPEAN PATENT APPLICATION

(11) **EP 0 887 050 A2**
(43) Date of publication of application: **30.12.1998**
(21) Application number: 98304972.7
(22) Date of filing: 24.06.1998
(51) Int. Cl.: A61C 15/04, A61K 7/16

(54) **Tartar control dental floss**

(30) Priority: 25.06.1997 US 882708
(71) Applicant: McNEIL-PPC, INC., Skillman, NJ 08558 (US)
(72) Inventor: Duden, Carol Anne, Lambertville, NJ 08530 (US); Harris, Michael Percival William, Belle Meade, NJ 08502 (US); Saindon, Mark David, Princeton, NJ 08540 (US)
(74) Representative: Mercer, Christopher Paul

(57) **Abstract**

The present invention is a dental floss comprising a dental floss substrate comprising at least one strand of polymeric material and an amount of a tartar control agent sufficient to retard the growth of tartar on at least one surface of human teeth. The tartar control agent comprises a salt of at least one alkali metal cation and at least one phosphorus-containing anion, preferably tetrasodium pyrophosphate. The phosphorus-containing anion is preferably present in the floss in an amount of at least about 7 mg per yard of floss, and more preferably, in an amount of at least about 9 mg per yard of floss. The tartar control agent is typically applied to the dental floss substrate with a coating composition comprising a coating polymer. The floss may further contain one or more additives such as flavorants, abrasives, lubricants, anti-caries agents, antimicrobials, antibiotics, antioxidants and desensitizers. The floss of the present invention was shown to retard the growth of tartar when used as part of a regular oral hygiene regimen.

## Description

### Field of the Invention

This invention relates to a dental floss that is effective in controlling tartar on the gingival regions of the teeth.

### Background

Calculus, or tartar as it is sometimes called, is a hard, mineralized formation that forms on the teeth at the gingival margin. Supragingival calculus appears principally in the areas near the orifices of the salivary ducts; i.e., on the lingual and facial surfaces of the lower anterior teeth, on the buccal surfaces of the first and second molars and on the distal surfaces of the posterior molars.

Mature calculus consists of an inorganic portion that is largely calcium phosphate arranged in a hydroxyapatite crystal lattice structure similar to bones, enamel and dentine. An organic portion is also present and consists of desquamated epithelial cells, leukocytes, salivary sediment, food debris and various types of microorganisms. It is formed on the teeth when crystals of calcium phosphate begin to be deposited in the pellicle and extracellular matrix of the dental plaque and become sufficiently closely packed together for the aggregates to become resistant to deformation. As the mature calculus develops, it becomes visibly white or yellowish in color unless stained or discolored by some extraneous agent. In addition to being unsightly and undesirable from an aesthetic standpoint, the mature calculus deposits are a constant source of irritation to the gingiva.

Methods to remove and/or control calculus generally fall into two categories. The first of these is mechanical calculus removal, which is best achieved by a dental physician using scaling instruments. Prevention may be partially achieved by conscientious daily oral hygiene (using tartar control dental products such as toothpaste and mouthwash) to remove plaque that forms each day before mineralization has occurred. However, inefficient brushing by most people leads to an accumulation of plaque (and ultimately calculus), particularly in the areas that are inaccessible by the toothbrush.

The chemical approach to calculus inhibition generally involves the use of chemical agents that chelate with calcium ion. Calcium ion chelation retards calculus either by inhibiting crystal growth, thereby preventing the calculus from forming, or by breaking down mature calculus by calcium ion removal.

The use of a dental floss containing a tartar control agent represents a tartar control means that combines both physical and chemical tartar control methods. Daily use of a dental floss containing an effective level of a tartar control agent such as the floss provided in accordance with the teachings of the present invention, as a part of a routine daily oral hygiene regimen, will insert the agent into the interproximal spaces between the teeth during use, thereby controlling tartar growth in these regions. Furthermore, after deposition between the teeth, the tartar control agent will subsequently diffuse to the gingival margins, thereby reducing tartar formation in these locations as well.

The dental floss prior art discloses numerous floss constructions and compositions that contain a tartar control agent, in one of numerous coatings.

U.S. 4,911,927 discloses a method of adding chemotherapeutic agents to dental floss. The method comprises the addition of a surfactant to the floss. The floss of that invention may contain chemotherapeutic agents at concentrations up to 40 mg/yd. The chemotherapeutic agents may include tetrasodium pyrophosphate. While this patent discloses numerous compositions that may be used to deposit active agents onto floss, only a single example (Example 49) discloses the use of tetrasodium pyrophosphate, in which the agent is present in the composition at a concentration of 5%. No disclosure is made, however, as to the amount of tartar-control agent actually included in the floss, or, for that matter, of the clinical efficacy of the resultant floss at reducing or controlling tartar.

U.S. 4,986,288 discloses a multi-filament dental floss containing a binding agent such as modified nylon resin, a wax coating, a coagulant, and, optionally, anticalculus or tartar control agents. The anti-tartar agents may include zinc chloride, and pyrophosphate salts such as tetrasodium, sodium acid and tetrapotassium pyrophosphates. This patent lists a desirable "formulation" as containing 3.3% pyrophosphate ion, 0.2% sodium fluoride and 1% of the polycarboxylate Gantrez. A floss referenced in that patent was said to release between 12 and 83% of the anti-tartar active during use. The broad range of release rates was attributed to the differing efficiencies with which the various subjects flossed. No disclosure is made, however, as to the amount of tartar-control agent included in the floss or of the clinical efficacy of the resultant floss in reducing or controlling tartar.

U.S. 5,033,488 discloses a dental floss comprising expanded polytetrafluoroethylene coated with microcrystalline wax. The floss may have incorporated thereon actives or agents such as coagulants, tartar control actives, and/or antibiotics. In regard to tartar control actives, a desirable active is said to be pyrophosphate, preferably 3.3% pyrophosphate ion; sodium fluoride, preferably 0.2%; and the polycarboxylate Gantrez, preferably 1%. The release of the tartar-control actives into the oral cavity from a microcrystalline wax matrix is expected to be relatively inefficient due to the insolubility of the matrix in the aqueous environment of the mouth. Once again, no disclosure is made as to the amount of tartar-control agent included in the floss or of the clinical efficacy of the resultant floss in reducing or controlling tartar.

U.S. 5,098,711 discloses a method of treating the oral cavity with multi-filament dental floss. The floss contains a cleaning preparation comprising a surfactant and a coating substance, and optionally, up to about 50% by weight of an active chemotherapeutic agent selected from antimicrobials, antibiotics, antioxidants, desensitizers and anti-tartar agents. Once again, only a single example (Example 49) of a cleaning preparation containing the tartar control agent tetrasodium pyrophosphate is provided, containing the agent at a concentration of 5%. No disclosure is made as to the amount of tartar-control agent included in the floss or of the clinical efficacy of the resultant floss in reducing or controlling tartar.

U.S. 5,165,913 discloses a dental floss containing a cleaning preparation, and optionally, further containing up to about 50% by weight of an active chemotherapeutic agent selected from antimicrobials, antibiotics, antioxidants, desensitizers, and anti-tartar agents. Chemotherapeutic removal of supergingival plaque is thought to be achieved by a combination of chemical cleansing by surfactants, modification of the surface of the plaque by coating materials, and alteration of the plaque with active ingredients including tetrasodium or tetrapotassium pyrophosphate. Once again, only a single composition containing the tartar control agent tetrasodium pyrophosphate is provided (Example 49), containing the agent at a concentration of 5%. No disclosure is made as to the amount of tartar-control agent included in the floss or of the clinical efficacy of the resultant floss in reducing or controlling tartar.

U.S. 5,209,251 discloses a dental floss made of expanded polytetrafluoroethylene in which the floss is coated with a wax to increase its coefficient of friction. In a preferred embodiment, the floss contains a bioactive component that is deposited either prior to or simultaneous with the deposition of the wax coating. The bioactive agent may include an anti-tartar agent, and may be selected from tetrasodium, disodium acid and tetrapotassium pyrophosphate. When a dentifrice is included in the floss, it is present at a concentration sufficient to provide a topical concentration of 5 to about 1000 ppm at the tooth surface. No disclosure is made as to the amount of tartar-control agent included in the floss or of the clinical efficacy of the resultant floss in reducing or controlling tartar.

U.S. Patent 5,503,842 discloses a polytetrafluoroethylene floss having a coating thereon. The coating is deposited on the floss from an emulsion containing polyvinyl alcohol and polyethylene glycol. The coating may contain a medicament and/or a flavorant. The coating may contain an anticalculus agent. Such anticalculus agents include the linear molecularly dehydrated alkali metal or ammonium salts such as sodium hexametaphosphate, sodium tripolyphosphate, disodium diacid phosphate, trisodium monoacid phosphate, tetrasodium polyphosphate and polyphosphates having the general formula of (NaPO₃)ₙ, where n is 2 to 125. It is said that this polyphosphate and other phosphates are preferably used in conjunction with synthetic anionic linear polymeric polycarboxylates. When present in the coating composition, an anti-calculus agent will be present in an amount of about 0.5 to about 10 percent by weight. In Example 1, a polytetrafluoroethylene substrate was coated with a composition comprising polyvinyl alcohol, polyethylene glycol 4000, sodium fluoride, glycerine, Gantrez S-97 polycarboxylate resin, tetrasodium polyphosphate and water to provide a floss said to contain 0.29% by weight of the polyphosphate. The patent does not indicate the clinical efficacy of this floss in reducing or controlling tartar.

While the above-cited patents disclose that dental floss may contain anti-tartar agents such as pyrophosphate salts, none of the prior art provides guidance as to the level of anti-tartar agent that must be incorporated into the floss to provide clinically proven efficacy during use.

It is an object of the present invention to provide a dental floss that is clinically effective at controlling tartar.

It is a further object of the present invention to provide a dental floss with a clinically effective amount of tartar control agent that is readily released from the floss in aqueous environments.

It is a further object of the present invention to provide a method for treating the oral cavity with a dental floss that is clinically proven to be effective at controlling tartar.

### Summary of the Invention

The present invention is a dental floss comprising a dental floss substrate comprising at least one filament of polymeric material and an amount of a tartar control agent sufficient to retard the accumulation or growth of tartar on at least one surface of human teeth. More particularly, the tartar control agent comprises a salt having at least one alkali metal cation and at least one phosphorus-containing anion. Hexametaphosphate, trimetaphosphate, tripolyphosphate, pyrophosphate and mixtures thereof are examples of phosphorus-containing anions present in the tartar control agents used in the floss of the present invention. The preferred phosphorus containing tartar control agent is tetrasodium pyrophosphate. The phosphorus-containing anion is preferably present in the floss in an amount of at least about 7 mg per yard of floss, and more preferably, in an amount of at least about 9 mg per yard of floss. The tartar control agent is preferably at least partially water soluble, and is preferably readily water extractable upon immersion of the floss in water.

The floss of the invention may be made from any of the common dental floss substrate materials such as polyamides, fluorinated polymers such as polytetrafluoroethylene, rayon, polyesters, acetate polymers, polyolefins, cotton, wool, silk and mixtures thereof. Polyamides such as nylon 6 and nylon 6,6 are preferred dental floss substrate materials.

The dental floss substrate used to prepare the floss of the present invention may be texturized.

The floss may further contain a coating composition comprising a coating polymer that serves to affix the tartar control agent to the floss substrate. The coating polymer is preferably at least partially water soluble and has a weight average molecular weight from about 100 to about 20,000, and more preferably, about 800 to about 14,000. Examples of suitable coating polymers are polyethyleneglycol, polyoxyethylene-polyoxypropylene block copolymers and mixtures thereof. The coating composition is preferably applied to the floss material at a temperature above the melting temperature of the coating polymer.

The tartar control agent may be applied to the floss material either simultaneous with, subsequent to or prior to the application of the coating composition. In addition to the tartar control agent, the flosses of the present invention may also comprise one or more additives such as flavorants, abrasives, lubricants, anti-caries agents, antimicrobials, antibiotics, antioxidants and desensitizers.

The floss of the present invention preferably has a denier of about 700 to about 2500, and is typically made from a dental floss substrate having a denier of about 300 to about 1500.

Another aspect of the present invention is the use of the present floss to retard the growth of tartar when used as part of a regular oral hygiene regimen.

### Brief Description of the Drawings

Figure 1 is a schematic view of an apparatus used to make the floss of the present invention.

Figure 2 is a schematic view of another apparatus that may be used in making the floss of the present invention.

### Detailed Description of the Invention

The present invention relates to a dental floss that is effective in controlling tartar on human teeth when used to floss the teeth. The dental floss of the present invention comprises a dental floss substrate comprising at least one filament of polymeric material and a tartar control agent in an amount sufficient to retard the growth of tartar on at least one surface of human teeth.

When the dental floss substrate comprises a single filament, the filament may have a circular or rectangular cross-section. In the case of a rectangular cross-section, the dimensions of the substrate are typically about 0.001 inches to about 0.010 inches in thickness and about 0.02 inches to about 0.12 inches in width. Dental flosses comprising a single filament of polytetrafluoroethylene, rectangular in cross-section, are well known in the art.

More typically, the dental floss substrate comprises a plurality of individual filaments which, if desired, may have a twist imparted thereto. A plurality of individual filaments may be grouped into strands, and one or more of these strands may be used as the dental floss substrate. When multiple strands are combined to form the dental floss substrate, a twist is typically incorporated into the dental floss substrate to prevent the strands from separating in processing and during use. The filaments may be straight or they may be texturized. The dental floss substrate, whether consisting of one filament or a plurality of filaments, may have any desired cross-sectional configuration. Typically, most dental flosses have a circular or rectangular cross-section. The flosses of the present invention may be made from dental floss substrate having a denier in the range of about 150 to about 1800. As mentioned earlier, the dental floss of the present invention is made from a dental floss substrate typically having a denier of about 300 to about 1500. Preferably, the dental floss substrate has a denier in the range of about 700 to about 1400. The dental floss of the present invention encompasses all of the above-mentioned embodiments.

The polymer that the dental floss substrate is comprised of may be any polymer typically used in the construction of dental floss. For example, the dental floss substrate may be comprised of polyamides, fluorinated polymers such as polytetrafluoroethylene, rayon, polyesters, acetate polymers, polyolefins, cotton, wool, silk and mixtures of these polymers. Preferably, the dental floss substrate is comprised of polyamide, and more preferably, the polyamide nylon 6 or nylon 6,6.

The floss of the present invention contains a tartar control agent in an amount sufficient to retard the growth of tartar on the teeth. The tartar control agent typically comprises a salt containing at least one alkali metal cation and at least one phosphorus-containing anion. Examples of phosphorus-containing anions useful in the present invention include hexametaphosphate ion, trimetaphosphate ion, tripolyphosphate ion, pyrophosphate ion and mixtures of these ions. Examples of salts useful in the present invention are tetrasodium pyrophosphate, tetrapotassium pyrophosphate and disodium dihydrogen pyrophosphate. A preferred tartar control agent for use in the floss of the present invention is tetrasodium pyrophosphate. It will be recognized that the use of disodium dihydrogen pyrophosphate, in which two of the four sodium ions of tetrasodium pyrophosphate are replaced by hydrogen ions, is also within the scope of the tartar control agents useful in the floss of the present invention. Similarly, other salts in which one or more of the alkali metal cations are replaced by hydrogen ions are also within the scope of the invention, provided that the salt contains at least one alkali metal cation. Likewise, the tartar control agent may contain different alkali metal cations, as would be the case with disodium dipotassium pyrophosphate. Alternatively, the tartar control agent may contain one or more alkali metal cations in conjunction with one or more alkaline earth cations. Such materials are also within the scope of the present invention.

The prior art known to us is silent on the amount of tartar control agent which must be present in a dental floss to impart a demonstrable retardation of tartar growth on human teeth. As will be shown subsequently, in the case of phosphorus-containing tartar control agent salts, the floss of the present invention should contain at least about 7 mg/yd of phosphorus-containing anion, and more preferably, at least about 9 mg/yd of phosphorus containing anion in order to provide tartar retardation. The floss of the present invention may contain up to about 20 to about 30 mg of phosphorus containing anion per yard of floss.

The floss of the present invention is believed to be effective by depositing the tartar control agent on the interproximal surfaces of adjacent teeth during flossing. For maximum effectiveness at controlling tartar at and above the gingival margin, it is desirable that any solid tartar control agent that may deposited between the teeth during flossing be at least partially water soluble in order to dissolve in the saliva and to be redistributed to the gingival regions during and after flossing. By partially water soluble, we mean that the agent should have a water solubility of at least about 1% at physiological temperature, approximately 37°C. Preferably, the agent should have a water solubility of at least about 2%, and more preferably, at least about 4% at physiological temperature.

The flosses of the present invention are preferably made by applying a coating composition to the dental floss substrate. The coating composition comprises a mixture of the tartar control agent, a coating polymer and, optionally, adjuvants such as sweeteners, flavorants, abrasives, lubricants, anti-caries agents, antimicrobials, antibiotics, antioxidants and desensitizers. The coating composition may be prepared by melting the coating polymer and adding the tartar control agent and any optional adjuvants. The coating composition is maintained above the melting point of the coating polymer to facilitate the application of the coating composition to the dental floss substrate. The coating composition may be applied to the dental floss substrate by unwinding a spool of the dental floss substrate and passing it through a tank containing the coating composition. The coating composition is allowed to solidify on the dental floss substrate after the coated dental floss substrate exits the coating tank, and the floss, coated with the coating composition containing the tartar control agent, is rewound onto a finished product spool.

Alternatively, the floss of the present invention may be made by coating the dental floss substrate with a coating composition containing the molten coating polymer absent any tartar control agent. Prior to cooling and solidification, the coated floss may be passed through or dusted with powdered tartar control agent, whereby the agent would then become embedded in the molten coating composition. As yet another alternative method of making the floss of the present invention, the dental floss substrate may be traversed through powdered tartar control agent to pick up the agent as a powder dispersed within the dental floss substrate. The agent may then be "fixed" onto the floss by over-coating with the coating composition containing the coating polymer.

To permit the tartar control agent to migrate from its initial points of deposition on the teeth to the gingival margin during and after flossing, the coating polymer should also be at least partially soluble in water to permit dissolution in the saliva. The coating polymer preferably has a water solubility of at least about 1% by weight, more preferably at least about 2% by weight, and most preferably, at least about 4% by weight at physiological temperature. Suitable water soluble polymers useful as coating polymers include the polyethyleneglycols, polyoxyethylene-polyoxypropylene block copolymers and mixtures of these polymers.

The coating polymers preferably have a weight average molecular weight of about 100 to about 20,000, and more preferably, from about 800 to about 14,000. The coating polymer should have a sufficiently high melting point so that it will solidify on the floss prior to re-rolling the coated floss on a take-up spool.

PEG 32, sold under the tradename Carbowax PEG 1450 by Union Carbide Corporation of Charleston, West Virginia, and Poloxamer 407, sold under the tradename Pluronic F-127 by BASF of Edison, New Jersey are exemplary polyethyleneglycol and polyoxyethylene-polyoxypropylene block copolymers, respectively, which are useful as coating polymers for the flosses of the present invention. Carbowax PEG 1450 conforms generally to the formula

HO (CH₂CH₂O)ₙ H

wherein n has an average value of 32. It has a weight average molecular weight of 1450, a melting point of 47°C, a viscosity of 30 centiStokes at 99°C and a solubility in water of 70% by weight at 20°C. Pluronic F-127 conforms generally to the formula 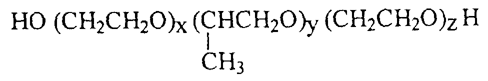 wherein x, y and z have average values of 98, 67 and 98, respectively. Pluronic F-127 has a weight average molecular weight of 12,600, a melting point of 56°C, a viscosity of 145 poise at 60°C and a solubility in water of 10% by weight at a temperature of 20°C.

The floss of the present invention may also contain one or more additional additives such as sweeteners, flavorants, abrasives, lubricants, anti-caries agents, antimicrobials, antibiotics, antioxidants and desensitizers. For example, the floss may contain sodium fluoride as an anti-caries agent at a concentration of about 0.05 to about 2 mg per yard, preferably at a concentration of about 0.1 to about 1 mg per yard, and most preferably, at a concentration of about 0.2 to about 0.5 mg per yard.

After coating with the tartar control agent and coating composition, the floss has a denier of about 700 to about 2500.

Not intending to be limited thereby, the following examples serve to illustrate the dental floss of the present invention.

The dental floss containing a tartar control agent of the present invention was made from a dental floss substrate consisting of strands of multifilament nylon 6,6 fiber. Each strand contained 68 filaments and had a denier of 200. Each strand was thermomechanically texturized, and four such strands were combined and were twisted at a frequency of about one twist per linear inch. Thus, the dental floss substrate contained 272 filaments and had a denier of 800. The dental floss substrate exhibited a breaking force of 8.8 lb and an elongation at break of 33%.

### Example 1 - Preparation of intermediate floss material

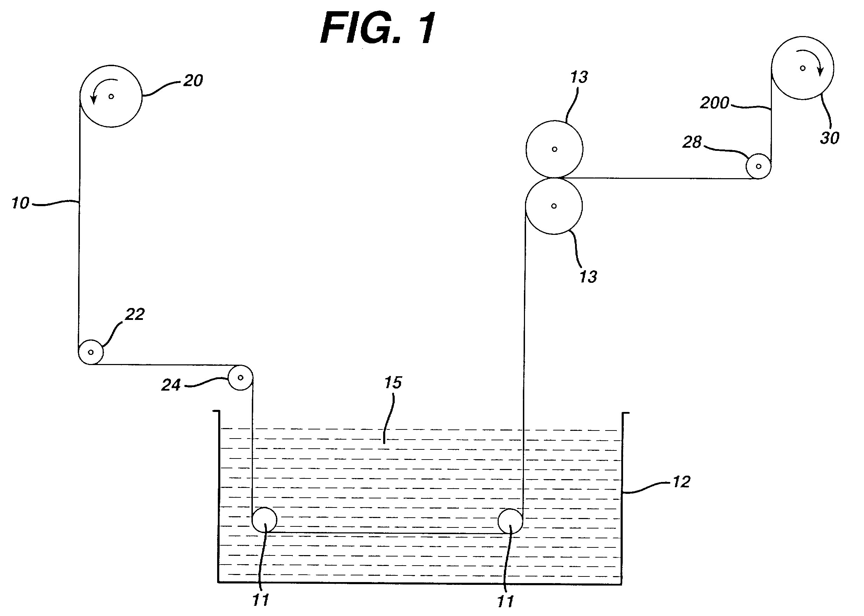

An intermediate floss material was formed by coating the above-mentioned dental floss substrate with the coating composition shown in Table 1 and using the apparatus shown in Figure 1. Dental floss substrate 10 was led from supply roll 20, passed under idler roller 22, over idler roller 24 into coating tank 12 containing coating composition 15. Floss material 10 was passed under spaced-apart splay bars 11,11 which function to spread the filaments apart while they passed through the coating tank. The coating composition was maintained at a temperature of 93°C, at which temperature the composition was in its molten state. After emerging from the coating tank, the coated substrate was passed between a pair of heated calender rollers 13,13 maintained at a temperature of about 86°C and spaced at a distance of 0.005 inches to force the coating composition 15 into the floss substrate and to remove excess coating. After passing between rolls 13,13, the coated floss substrate passed under roller 28 and was then re-wound onto take-up roll 30 as intermediate floss material 200. Under these conditions, a total of 90.7 mg of coating composition was added per yard of dental floss substrate. It will be understood that coating composition 15 solidified during travel of the coated floss substrate from calender rolls 13,13 to idler roll 28. If necessary, cooled air may be applied to the coated floss substrate during such travel to enhance the solidification process. It will be obvious to one skilled in the art that the add-on of the coating composition and the degree of uniformity of the coated material are influenced by such variables as the degree of splaying of the dental floss substrate, the line speed, tension and twist of the floss material, and such variables as the viscosity and temperature of the coating composition.

**Table 1**

| Tradename | Source | Chemical Description | Percentage of component in composition (weight percent) |
|---|---|---|---|
| Pluronic F-127 | BASF, Edison NJ | Polyoxyethylene-polyoxypropylene block copolymer | 77.4 |
| Antifoam 1500 | Dow Corning, Midland, MI | Liquid silicone compound | 10.0 |
| Insoluble saccharin | PMC Specialties Cincinnati, OH | 1,2-benzisothiazolin-3-one-1,1-dioxide | 2.3 |
| Tenox | Eastman Chemical, Kingsport, TN | Propyl Gallate | 0.1 |
| Versene (R) Acid Chelating Agent | Dow Chemical Midland, MI | Ethylenediamine-tetraacetic acid | 0.2 |
| Sident 10 | Degussa Corp., Ridgefield Park, NJ | Silica | 4.0 |
| Flavor | | | 6.0 |

### Comparative Example 1 and Examples 2-4 - Preparation of dual coated floss material

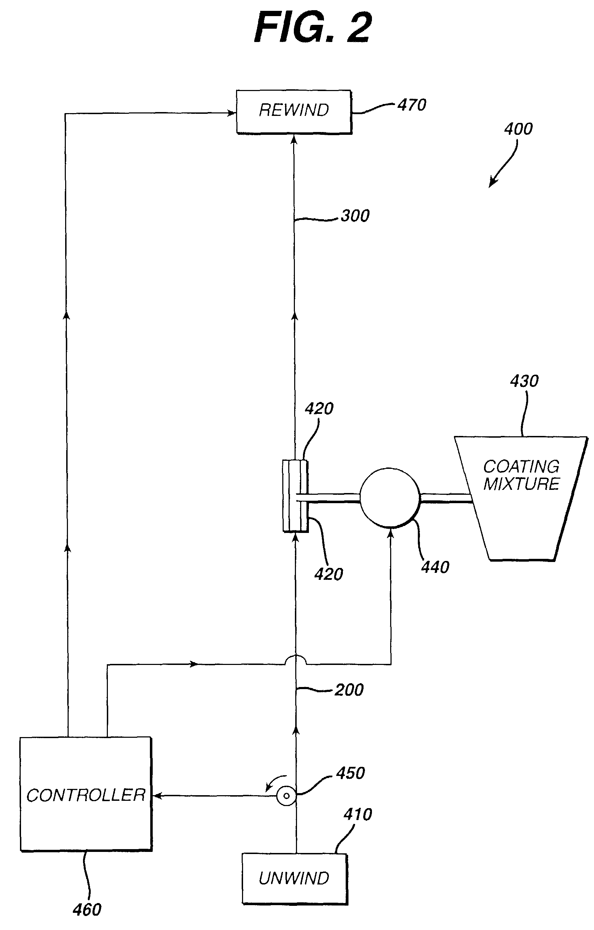

Intermediate floss 200 made in the manner set forth in Example 1 was coated a second time with the coating compositions shown in Table 2. The second coating process was conducted using the coating system 400 illustrated in Figure 2 of the drawings. Intermediate floss material 200 was let off from unwind roll 410 and passed through die station 420 where the coating composition was applied uniformly and continuously to its exterior surface. Die station 420 includes a V-shaped groove (not shown in the drawings) through which intermediate floss 200 passes. An opening (not illustrate in the drawings) is provided at the base of the V-shaped groove for delivering the coating composition in its molten state to the base of the groove. The aforementioned opening in die station 420 is coupled to a heated supply tank 430 by a coating pump 440. Heated supply tank 430 maintains the molten coating composition contained therein at its desired temperature. As the overcoated floss exits die station 420, the molten coating composition cools and solidifies and the finished floss 300 is then rewound onto a take-up roll at rewind station 470.

A velocity sensor 450 is provided for monitoring the velocity of floss 200 passing through system 400. The output of velocity sensor 450 is coupled to a controller 460. Controller 460 is also coupled to and provides control signals to pump 440 and to rewind station 470. The control signal provided to pump 440 ensures that, for a given length of intermediate floss 200 passing through die station 420, a constant and controlled amount of the coating composition is delivered to die station 420 and applied uniformly along a given length of floss. The control signal provided to rewind station 470 ensures that finished floss 300 is wound onto the take-up roll at the same rate that intermediate floss 200 is unwound at station 410. Finally, a tensioner (also not shown) is provided for maintaining a tension of about 10-150 grams in the floss as it moves from unwind station 410 to rewind station 470.

**Table 2**

| Example # | Comparative Example 1 | Example 2 | Example 3 | Example 4 |
|---|---|---|---|---|
| Carbowax PEG 1450 (weight % in coating composition) | 100 | 91.5 | 81.5 | 74.5 |
| Tetrasodium pyrophosphate (weight % in coating composition) | 0 | 8.5 | 18.5 | 25.5 |
| Coating add-on (mg/yd) | 61.3 | 58.4 | 59.0 | 59.4 |
| Add-on of tetrasodium pyrophosphate (mg/yd) | 0 | 4.0 | 10.0 | 14.0 |
| Add-on of pyrophosphate ion (mg/yd) | 0 | 2.62 | 6.54 | 9.16 |

### Comparative Example 2 and Example 5 - Preparation of Tartar Control Floss via a single coating

The dental floss substrate was coated with the compositions of Table 3 using the apparatus shown in Figure 1 and under the conditions of Example 1.

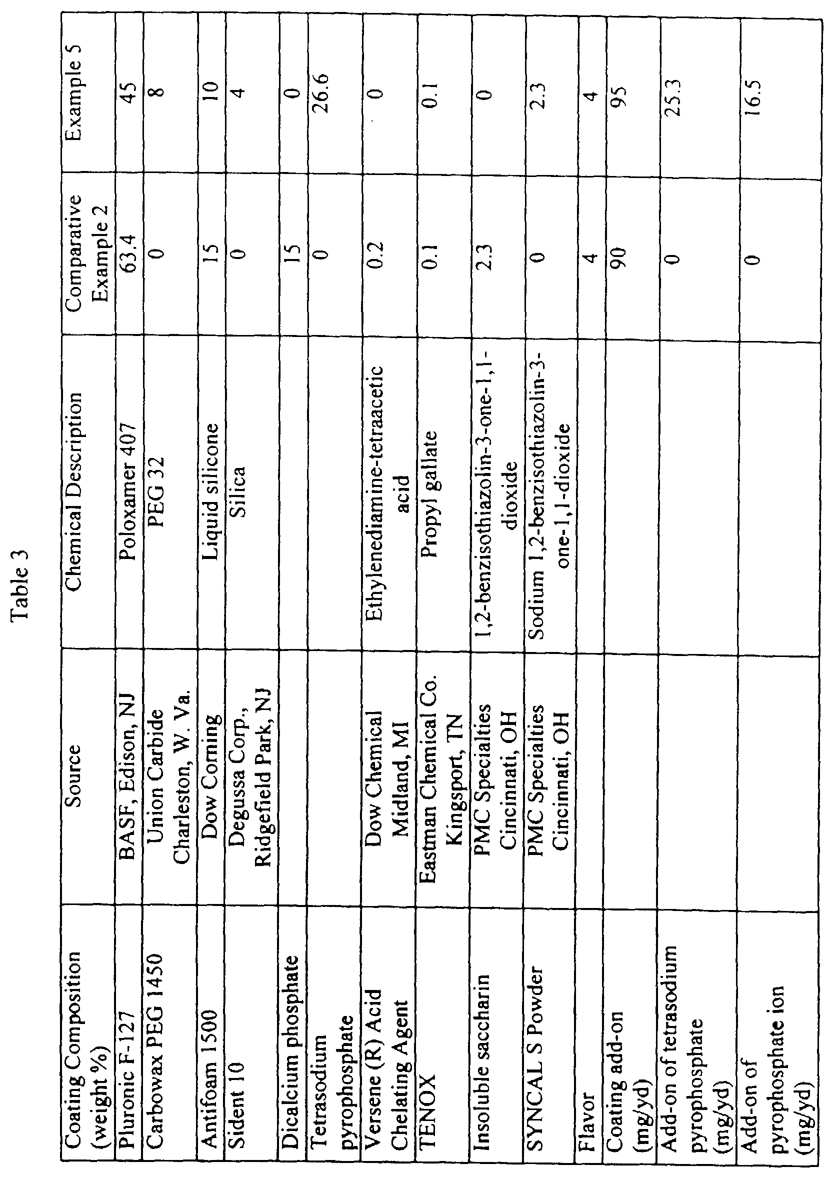

### Amount of Water-Soluble Tartar-Control Agent

In use, the tartar control agent in its coating matrix will be transferred from the floss into the interproximal spaces between the teeth. Once deposited on and in between the teeth, optimal effectiveness of the tartar control agent is expected when the agent would be solubilized in aqueous media of the type present in the oral cavity. The amount of water-extractable tartar control agent contained in the floss was measured as a function of time as follows:

The quantitative method for the determination of tetrasodium pyrophosphate is based on the assay method described in the Food Chemicals Codex 4^{th} edition, National Academy Press, Washington D.C., 1996, p. 378. The floss to be tested was cut into three-yard lengths. For each test, eight of these three-yard lengths were placed in a 250 mL, beaker. 100 mL of deionized water was added, and the beaker contents were stirred for the extraction times shown in Table 4. The floss was removed from the beaker, and excess liquid was expressed from the floss back into the beaker. The pH ofthe extract was adjusted to 3.8, 50 mL of 12.5% zinc sulfate heptahydrate solution was added, and the resultant solution was stirred for an additional two minutes. The solution was then titrated with 0.1 N sodium hydroxide solution to a pH of 3.8. Each mL of 0.1 N sodium hydroxide solution is equivalent to 13.3 mg of tetrasodium pyrophosphate.

Samples of floss prepared according to Examples 4 and 5 were tested by the above-referenced method. To determine the speed with which the tetrasodium pyrophosphate is extracted from the floss samples, the solutions were analyzed after contacting the floss for 30 seconds, 2 minutes and 5 minutes. For comparison, a commercially available dental floss, sold under the name of Colgate Total™ Tartar Control and distributed by Colgate Oral Pharmaceuticals, Inc. of Canton, MA, was also analyzed by the above-referenced method. The package label for the Colgate product states that it contains tetrasodium pyrophosphate. The results of these analyses are shown in Table 4.

**Table 4**

| | Amount of tetrasodium pyrophosphate extracted (mg/yd; % added) | | |
|---|---|---|---|
| Extraction time | 30 seconds | 2 minutes | 5 minutes |
| Floss of Example 4 | 11.1mg; 79.3% | 11.8mg; 84.3% | 12.5mg; 89% |
| Floss of Example 5 | 24.9mg; 98.6% | 25.4mg;100.3% | 29.9mg;118% |
| Colgate Total Tartar Control Floss | 0 | 0 | 0 |

Table 4 shows the amount of tetrasodium pyrophosphate extracted, both in mg/yd as well as a percentage of the amount contained in the floss. The data indicate that for the floss of the present invention, at least 75% of the amount of tetrasodium pyrophosphate contained in the floss is solubilized in aqueous media within 30 seconds. Extraction amounts greater than 100% in Table 4 are attributed to sample variations due to variations within the coating process. In contrast, the commercially available Colgate product shows no detectable soluble pyrophosphate, even after 5 minutes of extraction time. These results point to the expected superiority of the floss of the present invention at controlling tartar relative to prior art flosses.

To test the actual amount of tetrasodium pyrophosphate released from the floss during flossing, samples of the floss of Example 5 were analyzed by 5 minute extraction with subsequent work-up as described above, both before and after flossing. Two 18-inch samples were analyzed before flossing and two samples were analyzed after flossing. The average amount of tetrasodium pyrophosphate extracted from the unused floss was 20 mg/yd, while the average amount extracted from the used floss was about 7 mg/yd. The difference between these values, i.e., 13 mg/yd, is taken to be the amount removed from the floss during flossing.

### Clinical Evaluation of Tartar-Control Floss

In-vivo calculus was measured using the Volpe-Manhold Index (VMI) as described in *J. Periodontal Res.* **9**; Suppl 14: 35-36, 1974. A population of adult participants was assembled for the clinical evaluation of the tartar control floss of the present invention. Dental impressions of the anterior mandibular teeth were made for each of the subjects, and toothshields that covered their six mandibular anterior teeth were constructed based on these impressions. A two-week pre-trial period was commenced with a thorough dental cleaning, after which the participants were instructed to brush their teeth twice daily with their toothshields in place. The toothshields were removed following brushing. After the two-week pre-trial period, the subjects were re-examined and those with a total VMI calculus score of 8 mm or more were accepted into the trial period. The tartar accumulations from the subjects' lower front teeth were removed by dental hygienists following the re-examinations. The subjects were next subjected to a washout period, in which they were instructed to brush according to their daily regimen without using their toothshields. A total of 60 subjects were then randomly assigned to 4 equivalent groups balanced by baseline total VMI score, gender and flossing habits. The test teeth were scaled and polished by hygienists to remove plaque and calculus, and the subjects were then issued their test floss for a two-week trial period. During the trial period, the subjects were told to brush twice daily with their toothshields in place and to then floss with their assigned test floss after removing their toothshields. Three flosses were tested, i.e., the flosses of Examples 2 through 4, the results of which were compared against the results of users of the floss of Comparative Example 1. At the end of the two-week trial period, the subjects were once again assessed for calculus and oral soft tissue health. The calculus scores of the various test groups, after log transformation, are shown in Table 5.

The data in Table 5 show the mean calculus scores of the subjects using the various test flosses. The calculus scores were separately determined for and are listed as a function of the various tooth surfaces. The labial calculus score is a summation of the gingival, distal and mesial calculus measurements over the labial surfaces of the six mandibular anterior teeth. The lingual calculus score is the summation of the gingival, distal and mesial calculus measurements over the lingual surfaces of the six mandibular anterior teeth. The proximal calculus score is the summation of the distal and mesial measurements over the lingual and the labial surfaces of all six mandibular anterior teeth. The gingival calculus score is the summation of the gingival measurements from the lingual and labial surfaces of all six mandibular anterior teeth. Calculus reductions are reported relative to the control group that used the floss of Comparative Example 1. Statistical significance of calculus reduction was determined at the 95% confidence level. As indicated in Table 5, the floss of Example 4, containing 14 mg/yd of tetrasodium pyrophosphate, exhibited a 31.7% reduction in calculus from the labial tooth surface and a 20% calculus reduction from the proximal tooth surface. The labial calculus reduction was statistically significant at the 98% confidence level, while the proximal reduction was only directional, not being statistically significant at the 95% confidence level.

**Table 5**

| | | VMI Calculus Scores | | |
|---|---|---|---|---|
| Floss of | Tooth Surface | Adjusted Mean ± SE¹ | Reduction (%)² | Significance³ |
| Comp. Ex. | Labial | 6.3 ± 1.2 | --- | --- |
| Ex. 2 | Labial | 5.5 ± 1.1 | 13 | ns |
| Ex. 3 | Labial | 6.7 ± 1.1 | -6 | ns |
| Ex. 4 | Labial | 4.0 ± 1.2 | 36.5 | (p<0.02) |
| | | | | |
| Comp. Ex. 1 | Lingual | 9.5 ± 1.1 | --- | --- |
| Ex. 2 | Lingual | 11.2 ± 1.1 | -18 | ns |
| Ex. 3 | Lingual | 9.7 ± 1.1 | -2 | ns |
| Ex. 4 | Lingual | 9.2 ± 1.1 | 3 | ns |
| | | | | |
| Comp. Ex. 1 | Proximal | 12.0 ± 1.1 | --- | --- |
| Ex. 2 | Proximal | 12.6 ± 1.1 | -5 | ns |
| Ex. 3 | Proximal | 12.4 ± 1.1 | -3 | ns |
| Ex. 4 | Proximal | 9.6 ± 1.1 | 20 | ns |
| | | | | |
| Comp. Ex. 1 | Gingival | 4.3 ± 1.1 | --- | --- |
| Ex. 2 | Gingival | 4.8 ± 1.1 | -12 | ns |
| Ex. 3 | Gingival | 3.9 ± 1.1 | 9 | ns |
| Ex. 4 | Gingival | 4.1 ± 1.1 | 5 | ns |

| | | | | |
|---|---|---|---|---|
| ¹ Covariate-adjusted total VMI score ± Standard Error of adjusted mean (in total mm). | | | | |
| ² Percent reduction relative to Comparative Example 1. | | | | |
| ³ Statistical differences between groups according to analysis of covariance using the pre-trial scores as covariates; ns = not statistically different at the 95% confidence level. | | | | |

A second clinical study was performed, comparing the efficacy of the floss of Example 5 relative to a control consisting of users of the floss of Comparative Example 2 containing no tetrasodium pyrophosphate. In this case, a total of 86 subjects divided into two groups were evaluated; half were given the floss of Example 5 and half were given the control. During the two-week pre-trial period, the participants were instructed to brush their teeth twice daily with their toothshields in place. The toothshields were removed following brushing, and all participants were instructed to floss their mandibular anterior teeth twice daily using the floss of Comparative Example 2. The evaluation protocol and hygiene regimen for the rest of the study were otherwise identical to that described above. The calculus scores of the test groups, after log transformation, are shown in Table 6. As shown in Table 6, the group using the floss of Example 5, containing 25 mg/yd of tetrasodium pyrophosphate, experienced a 37% statistically significant reduction in labial calculus and a 21% statistically significant reduction in proximal calculus relative to the control group using the floss of Comparative Example 2. The labial and proximal calculus reductions were statistically significant at the 99.9% and 97% confidence levels, respectively. Furthermore, oral soft tissue observations demonstrated that there were no relevant pathologic conditions or adverse reactions attributable to the use of any of the flosses of these studies.

**Table 6**

| | | VMI Calculus Scores | | |
|---|---|---|---|---|
| Floss of | Tooth Surface | Adjusted Mean ± SE¹ | Reduction (%)² | Significance³ |
| Comp. Ex. 2 | Labial | 6.3 ± 1.1 | --- | --- |
| Ex. 5 | Labial | 4.0 ± 1.1 | 37 | (p<0.001) |
| | | | | |
| Comp. Ex. 2 | Lingual | 10.1 ± 1.1 | --- | --- |
| Ex. 5 | Lingual | 9.8 ± 1.1 | 3 | ns |
| | | | | |
| Comp. Ex. 2 | Proximal | 12.6 ± 1.1 | --- | -- |
| Ex. 5 | Proximal | 10.0 ± 1.1 | 21 | (p<0.03) |
| | | | | |
| Comp. Ex. 2 | Gingival | 4.1 ± 1.1 | --- | --- |
| Ex. 5 | Gingival | 4.4 ± 1.1 | -7 | ns |

| | | | | |
|---|---|---|---|---|
| ¹ Covariate-adjusted total VMI score ± Standard Error of adjusted mean (in total mm). | | | | |
| ² Percent reduction relative to Comparative Example 2. | | | | |
| ³ Statistical differences between groups according to analysis of covariance using the pre-trial scores as covariates; ns = not statistically different at the 95% confidence level. | | | | |

While particular embodiments of the present invention have been illustrated and described herein, the present invention should not be limited by such illustrations and descriptions. It should be apparent that changes and modifications may be incorporated and embodied as part of the present invention within the scope of the following claims.

## Claims

1. A dental floss comprising:
a. a dental floss substrate comprising at least one filament of polymeric material; and
b. an amount of a tartar control agent sufficient to retard the growth of tartar on at least one surface of human teeth.

2. The dental floss of claim 1 wherein the tartar control agent comprises a salt comprising at least one alkali metal cation and at least one phosphorus-containing anion.

3. The dental floss of claim 2 wherein the phosphorus-containing anion is hexametaphosphate, trimetaphosphate, tripolyphosphate, pyrophosphate or a mixture thereof and is preferably pyrophosphate.

4. The dental floss of claim 2 or claim 3 wherein the salt comprises tetrasodium pyrophosphate.

5. The dental floss of any one of claims 2 to 5 wherein the phosphorus-containing anion is present in the floss at a level of at least about 7mg per yard of floss, preferably at least about 9mg per yard of floss, more preferably at most about 30mg per yard of floss and most preferably at most about 20mg per yard of floss.

6. The dental floss of any one of claims 1 to 5 wherein the tartar control agent is at least partially water soluble.

7. The dental floss of any one of claims 1 to 6 wherein a majority of the tartar control agent is readily extractable upon immersion of the floss in water, preferably wherein at least 75 percent of the tartar control agent contained in the floss is extracted into water within 5 minutes of being immersed in water.

8. The dental floss of any one of claims 1 to 7 wherein said at least one filament of polymeric material comprises:
a polyamide such as nylon 6, nylon 6,6 or a mixture thereof; a fluorinated polymer such as polytetrafluoroethylene; rayon; polyester; an acetate polymer; a polyolefin; cotton; wool; silk; or a mixture thereof.

9. The dental floss of any one of claims 1 to 8 wherein said dental floss substrate comprises a plurality of said filaments.

10. The dental floss of any one of claims 1 to 9 wherein said dental floss substrate is or at least some of said filaments are texturized.

11. The dental floss of any one of claims 1 to 10 which further comprises a coating composition comprising a coating polymer, said coating composition serving to affix the tartar control agent to the dental floss substrate.

12. The dental floss of claim 11 wherein the coating polymer is at least partially water soluble.

13. The dental floss of claim 11 or claim 12 wherein the coating polymer has a weight average molecular weight from about 100 to about 20,000, preferably from about 800 to about 14,000.

14. The dental floss of any one of claims 11 to 13 wherein the water soluble polymer is polyethyleneglycol, polyoxyethylene-polyoxypropylene block copolymer or a mixture thereof.

15. The dental floss of any one of claims 11 to 14 wherein the coating composition is applied to the dental floss substrate at a temperature above the melting temperature of the coating polymer.

16. The dental floss of any one of claims 11 to 15 wherein the tartar control agent is applied to the dental floss substrate prior to, simultaneously with or subsequent to the application of the coating composition.

17. The dental floss of any one of claims 1 to 16 which further comprises at least one additive which is a flavorant, abrasive, lubricant, anti-caries agent, antimicrobial, antibiotic, antioxidant or densensitizer.

18. The dental floss of any one of claims 1 to 17 wherein the dental floss substrate has a denier from about 150 to about 1800 or about 700 to about 2500.
